# FASCICULE DE BREVET EUROPEEN

(11) **EP 3 282 382 B1**
(45) Date de publication et mention de la délivrance du brevet: **29.05.2019**
(21) Numéro de dépôt: 17305908.0
(22) Date de dépôt: 11.07.2017
(51) Int. Cl.: G16H 50/70, A61B 5/00, A61M 16/00, A61B 5/08

(54) **SYSTÈME DE TRAITEMENT DE DONNÉES POUR PRÉDIRE UNE CRISE D'EXACERBATION D'UN PATIENT ATTEINT D'UNE MALADIE RESPIRATOIRE CHRONIQUE**
DATENVERARBEITUNGSSYSTEM ZUR VORHERSAGE EINER AKUTEN ZUSTANDSVERSCHLIMMERUNG EINES PATIENTEN, DER AN EINER CHRONISCHEN ATEMWEGSERKRANKUNG LEIDET
DATA-PROCESSING SYSTEM FOR PREDICTING AN EXACERBATION ATTACK OF A PATIENT SUFFERING FROM A CHRONIC RESPIRATORY DISEASE

(30) Priorité: 10.08.2016 FR 1657672
(43) Date de publication de la demande: 14.02.2018
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: AMADOU-BOUBACAR, Habiboulaye, 78350 Jouy en Josas (FR); TEXEREAU, Joëlle, 75013 Paris (FR)
(74) Mandataire: Pittis, Olivier

(56) Documents cités:
- WO-A1-2015/062811
- WO-A2-2008/112353
- FR-A1- 3 021 872
- US-A1- 2008 275 349
- MAS S. MOHKTAR ET AL: "Predicting the risk of exacerbation in patients with chronic obstructive pulmonary disease using home telehealth measurement data", ARTIFICIAL INTELLIGENCE IN MEDICINE, vol. 63, no. 1, 1 janvier 2015 (2015-01-01), pages 51-59, XP055318538, NL ISSN: 0933-3657, DOI: 10.1016/j.artmed.2014.12.003
- D. SANCHEZ-MORILLO ET AL: "Use of predictive algorithms in-home monitoring of chronic obstructive pulmonary disease and asthma: A systematic review", CHRONIC RESPIRATORY DISEASE, vol. 13, no. 3, 20 avril 2016 (2016-04-20) , pages 264-283, XP055317815, ISSN: 1479-9723, DOI: 10.1177/1479972316642365
- AINA M. YAÑEZ ET AL: "Monitoring Breathing Rate at Home Allows Early Identification of COPD Exacerbations", CHEST, vol. 142, no. 6, 1 décembre 2012 (2012-12-01), pages 1524-1529, XP055317810, US ISSN: 0012-3692, DOI: 10.1378/chest.11-2728

## Description

L'invention porte sur un système de traitement de données permettant de prédire une crise d'exacerbation d'un patient atteint d'une maladie respiratoire chronique, en particulier d'une BPCO, lequel patient est traité par oxygénothérapie, en particulier à son domicile, et d'alerter ensuite le personnel soignant ou analogue de manière à éviter ou à réduire la fréquence des ré-hospitalisations dudit patient, ainsi que sur une installation de suivi de patient incluant un tel système de traitement de données.

Les maladies respiratoires chroniques sont en forte progression dans le monde, en particulier la BPCO ou Broncho-Pneumopathie Chronique Obstructive qui affecte les broches des patients, par exemples chez les fumeurs. La BPCO est une maladie chonique dont l'évolution progressive est jalonnée de poussées d'aggravation des symptômes récurrents de la maladie, appelées crises d'exacerbations de BPCO ou plus simplement exacerbations, notamment une augmentation de la toux et de l'essoufflement.

La répétition des exacerbations aggrave le pronostic de la maladie, donc les risques vitaux du patient. De ce fait, en cas de crise aigüe de BPCO ou analogue, le patient est généralement hospitalisé pour être traité, notamment soigné par administration d'oxygène gazeux, également appelé oxygénothérapie.

Or, on comprend aisément qu'une telle hospitalisation engendre un processus lourd et coûteux, voire même parfois traumatisant pour le patient qui doit passer plusieurs jours en hôpital. Ensuite, après son hospitalisation, le patient rentre à son domicile où il doit généralement suivre un traitement par oxygénothérapie basé sur une administration d'oxygène gazeux, typiquement produit par un concentrateur d'oxygène ou de tout autre système de fourniture d'oxygène utilisable au domicile du patient.

Pour éviter ou minimiser la fréquence de rechutes et donc de hospitalisations des patients, il convient de pouvoir détecter le plus tôt possible la survenance d'une nouvelle crise de BPCO ou analogue chez les patients, c'est-à-dire de pouvoir prédire les exacerbations de BPCO de manière à permettre une prise en charge des patients avant une crise et éviter leur re-hospitalisation.

Or, ce n'est pas chose aisée, notamment du fait que les patients se trouvent à leur domicile et que l'on doit donc pouvoir détecter les crises d'exacerbations de BPCO rapidement et surtout à distance. Plusieurs documents se sont déjà attelés à ce problème et ont proposés des solutions qui se sont avérées en pratique rarement efficaces.

Ainsi, le document Chest; Dec. 2012;142(6):1524-9 ; Monitoring breathing rate at home allows early identification of COPD exacerbations; A.M. Yanez et al, propose de prédire les exacerbations de BPCO à partir de l'analyse de la fréquence respiratoire du patient. En effet, il a été montré que la fréquence respiratoire augmente chez 70% des 30 patients testés, durant les 5 jours précédant une exacerbation. Or, la fréquence respiratoire seule n'est pas suffisante pour rendre la prédiction fiable.

Par ailleurs, WO-A-2015062811 propose d'effectuer des mesures de débit et de pression du flux de gaz administré au patient pour en extraire une fréquence respiratoire qui est ensuite analysée. Un algorithme embarqué dans le capteur est chargé de détecter les exacerbations en utilisant la méthode de Young's C Statistic. Là encore, la fréquence respiratoire seule n'est pas suffisante pour rendre la prédiction fiable.

D'autres documents proposent d'exploiter d'autres paramètres.

Ainsi, US-A-2013/030258 propose une méthode de détection de l'exacerbation de BPCO basée sur des critères prédéfinis relatifs à des changements de fréquence respiratoire et de fréquence cardiaque en fonction de variations de l'activité physique du patient.

US-A-2013/0310699 propose une méthode basée sur le recueil de signaux mesurés par des électrodes placées sur les muscles thoraciques du patient, lesquels servent à déterminer différents paramètres de suivi, notamment les rythmes cardiaque et respiratoire, qui permettent de détecter les détériorations de la maladie.

US-A-2014/0221782 propose une méthode théorique utilisant le niveau d-saturation en oxygène dans le sang du patient mesuré par oxymétrie. Un algorithme de prédiction effectue une régression sur les données sur une fenêtre temporelle glissante de 30 jours pour estimer si le patient s'approche d'une exacerbation.

US-A-2014/0206949 décrit un système utilisant un ou plusieurs dispositifs, notamment un spiromètre, un oxymètre pulsé, un thermomètre et un capteur au peroxyde, mettant en oeuvre une méthode de prédiction se basant sur un réseau bayésien pour fournir une probabilité d'exacerbation. Le patient doit effectuer lui-même les mesures.

US-A-2011/0184250 enseigne de réaliser d'abord des mesures des conditions ambiantes dans l'environnement du patient, notamment qualité d'air, niveau d'allergènes, température et conditions atmosphériques. Un algorithme prédit la probabilité d'une exacerbation de BPCO en fonction de l'activité attendue pour le patient dans les conditions ambiantes locales, et alerte le patient des risques avant la période concernée.

On connait par ailleurs les documents suivants :
- Mas S. Mohktar et al; Predicting the risk of exacerbation in patients with chronic obstructive pulmonary disease using home telehealth measurement data; Artificial Intelligence in medicine, Vol. 63, N°1, 01 janvier 2015, p. 51-59*;* qui porte sur une méthode de prédiction des exacerbations de BPCO utilisable pour des patients traités à domicile, et
- US-A-2008/275349 propose un appareil comprenant un capteur configuré pour détecter un paramètre physiologique d'une personne ainsi que ses mouvements de corps. Ces paramètres sont traités et une alarme est déclenchée en cas de détérioration des conditions du suivi (monitorage).

On connait par ailleurs FR-A-3021872 qui enseigne un procédé et un dispositif de détection de l'aggravation de l'état cardio-respiratoire d'un patient, à partir de trois paramètres qui sont forcément toujours détectés, à savoir la fréquence respiratoire, le pourcentage de cycles déclenchés par le patient et la durée d'utilisation de l'appareil déterminés pendant trois fenêtres d'observation. Ensuite, on détecte des variations significatives de ces paramètres pendant au moins 2 jours sur une période de 3 jours ou plus. Si une variation significative est détectée, une alarme est déclenchée.

Toutefois, ces différentes solutions ne sont pas totalement satisfaisantes puisqu'elles ne permettent pas, elles non plus, de prédire de manière fiable et efficace, les exacerbations BPCO ou analogues chez les patients suivants un traitement d'oxygénothérapie, en particulier des patients traités à domicile.

Certaines de ces solutions ne permettent pas de suivre le patient à distance et/ou nécessitent que le patient réalise lui-même certaines mesures. Ce n'est pas pratique et engendre des risques d'erreur de mesure. D'autres ne sont pas concluantes lorsqu'on les met en oeuvre puisqu'on constate en pratique que beaucoup d'exacerbations ne sont pas détectées par ces méthodes. D'autres encore sont très théoriques et difficilement applicables au domicile d'un patient car trop contraignantes et/ou non-adaptées à un patient actif, c'est-à-dire qui déambule.

Au vu de cela, le problème qui se pose est dès lors de pouvoir détecter au plus tôt, c'est-à-dire prédire, une détérioration de l'état de santé d'un ou plusieurs patients atteints d'une maladie respiratoire, en particulier une BPCO, lesquels patients sont traités par oxygénothérapie à leur domicile, et en réponse à cette détection, d'alerter les professionnels de santé ou analogue pour qu'ils puissent prendre des mesures de traitement efficaces, le plus tôt possible, de manière à éviter ou à réduire la fréquence des ré-hospitalisations de ces patients dues aux exacerbations répétées de la BPCO ou analogue.

La solution de l'invention est alors un système de traitement de données permettant de prédire une crise d'exacerbation d'un patient atteint d'une maladie respiratoire chronique, en particulier d'une BPCO, et traité par oxygénothérapie, comprenant au moins un processeur configuré pour :
a) déterminer des variations de pression (DP) de flux de gaz pendant une durée donnée (dt), à partir de mesures de pression (P) d'un flux de gaz d'oxygénothérapie administré à un patient pendant la durée donnée (dt),
b) déterminer une durée de respiration (Dresp) du patient, avec Dresp < dt, pendant la durée donnée dt, à partir des variations de pression (DP) pendant ladite durée donnée (dt), ), ladite durée de respiration (Dresp) étant définie comme la durée, pendant la période de temps dt considérée, pendant laquelle le patient a inhalé du gaz, déduire des mesures de pression (P) au moins une valeur de fréquence respiratoire (Val_FR) pendant la durée donnée dt et répéter plusieurs fois cette détermination de valeur de fréquence respiratoire pendant la durée de respiration (Dresp) de manière à obtenir plusieurs valeurs de fréquences respiratoires (Val_FR) successives mesurées pendant la durée de respiration (Dresp),
c) calculer au moins une valeur moyenne de fréquence respiratoire (FR_moy) sur la durée de respiration (Dresp), à partir de plusieurs valeurs de fréquences respiratoires (Val_FR) successives mesurées pendant la durée de respiration (Dresp),
d) calculer au moins une valeur moyenne de durée de respiration (Vmoy_Dresp) à partir de plusieurs durées de respiration (Dresp) successives pendant une durée longue dL,
e) déterminer, à partir de plusieurs valeurs moyennes de fréquence respiratoire (FR_moy) obtenues sur la durée longue (dL), une valeur moyenne (Vmoy_FR) de fréquence respiratoire,
f) répéter tout ou partie des opérations opérées en a) à e) pour obtenir plusieurs valeurs moyennes de durée de respiration (Vmoy_Dresp) et plusieurs valeurs moyennes(Vmoy_FR) de fréquence respiratoire, sur une durée de plusieurs jours,
g) calculer à partir des valeurs moyennes de durée de respiration (Vmoy_Dresp) et des valeurs moyennes (Vmoy _FR) de fréquence respiratoire obtenues sur la durée de plusieurs jours, plusieurs variables statistiques (v₁, v₂, ..., vₘ) correspondant au moins aux valeurs :
   - de coefficient linéaire et de la somme des valeurs moyennes de durées de respiration (Vmoy_Dresp), et
   - de coefficient linéaire, de la valeur médiane, de la variance et de l'auto-corrélation estimées à partir des valeurs moyennes (Vmoy_FR) de fréquence respiratoire,
h) sélectionner au sein d'au moins une base de données, plusieurs variables représentatives du profil sociodémographique du patient mémorisées au sein de ladite au moins une base de données,
i) comparer à chaque date t prédéfinie (par exemple toutes les 24h), un vecteur multidimensionnel Xt, représentant au moins les variables statistiques (v₁, v₂, ..., vₘ) issues des données respiratoires et les variables (x₁, x₂, ..., xₙ) représentatives du profil sociodémographique du patient, à un ou plusieurs modèles mathématiques prédéfinis et mémorisés, le ou lesdits modèles mathématiques étant estimés à partir de données correspondant à des états de santé normaux et à des états pré-exacerbatoires, et
j) déduire de la comparaison avec le ou lesdits modèles mathématiques, l'état de santé du patient considéré de manière à prédire une exacerbation.

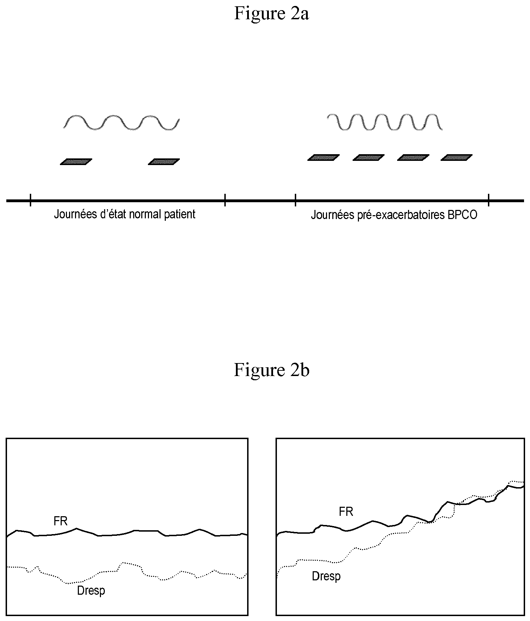

La fréquence respiratoires (FR) et de la durée de respiration (Dresp)sont des paramètres extrêmement importants dans le cadre de la présente invention. En effet, ce sont ces paramètres qui permettent de définir le vecteur multidimensionnel, en particulier tridimensionnel. En fait, la durée de respiration (Dresp) (i.e. le temps pendant lequel le patient a utilisé son masque par exemple pour respirer de l'oxygène), également appelée de durée de traitement est particulièrement importante dans la prédiction des exacerbations car, dans les jours qui précèdent une exacerbation, on assiste à une augmentation de l'utilisation de la source d'oxygène alimentant le patient, donc à une augmentation de la durée de respiration (Dresp). En effet, le patient commence à sentir les effets d'une insuffisance en oxygène et va en conséquence accroître sa durée de respiration (Dresp). Ceci est notamment illustré en Figure 2b.

Or, ceci n'avait jamais été mis en évidence dans l'état de l'art technique. Détecter les exacerbations répétées de la BPCO ou analogue, en utilisant en particulier le paramètre de durée de respiration (Dresp), constitue donc une avancée notable puisque ceci permet d'alerter au plus tôt le professionnel de santé ou analogue pour qu'ils puissent prendre des mesures de traitement efficaces et rapides pour éviter ou réduire la fréquence de ré-hospitalisation du patient considéré.

Selon le cas, le système de traitement de données de l'invention peut comprendre l'une ou plusieurs des caractéristiques techniques suivantes :
- ledit au moins un processeur est configuré pour créer au moins un vecteur multidimensionnel Xt à partir des variables statistiques (v₁, v₂, ..., vₘ) issues des données respiratoires Vmoy_Dresp et Vmoy_FR, et des variables (x₁, x₂, ..., xₙ) représentatives du profil sociodémographique du patient. Ce vecteur multidimensionnel Xt constitue, i.e. fournit, une représentation dynamique à l'état de santé du patient sur chaque durée longue dL.
- ledit au moins un processeur est configuré pour (b) déterminer plusieurs valeurs de fréquence respiratoire (Val_FR) sur des intervalles de temps (I₁, I₂, I₃, ... Iₙ) successifs pendant la durée de respiration (Dresp) de manière à obtenir plusieurs valeurs consécutives de fréquences respiratoires (Val_FR) successives mesurées pendant la durée de respiration (Dresp),
- les intervalles de temps (I₁, I₂, I₃, ... Iₙ) ont des durées entre 30 secondes et plusieurs minutes, par exemple des intervalles de 30 sec à 10 minutes, typiquement de 1 à 5 minutes.
- la durée de respiration (Dresp) correspond à la durée, pendant la période de temps dt considérée, pendant laquelle le patient a inhalé du gaz. La durée de respiration (Dresp) est donc formée d'une succession d'intervalles de temps pendant lesquels le patient a respiré du gaz. Plusieurs fréquences respiratoires FR peuvent donc être mesurées pendant ces intervalles de temps, donc pendant la durée totale Dresp, de manière à déterminer plusieurs valeurs de fréquence respiratoire (Val_FR), étant donné que la fréquence respiratoire FR n'est pas constante pendant la durée de respiration (Dresp).
- la partie A) b) est parfaitement claire car elle définit une durée « longue » dt pendant laquelle on détermine une durée plus courte Dresp appelée 'durée de respiration'. Par exemple, si la durée dt est égale à 1 heure, alors la durée Dresp peut être de 50 minutes seulement. En effet, pendant un traitement d'oxygénothérapie, l'appareil peut délivrer de l'oxygène (pendant une durée dt) mais le patient peut ne pas respirer cet oxygène car il a enlevé son masque respiratoire par exemple.
- ledit au moins un processeur est configuré pour émettre une alerte lorsque, pour un patient donné, une exacerbation est détectée, et de préférence pour afficher une information d'état de santé du patient.
- il comprend des moyens de mémorisation conçus pour mémoriser :
   - les variables statistiques (v₁, v₂, ..., vₘ) obtenues à partir des valeurs moyennes de durée de respiration (Vmoy_Dresp) et les valeurs moyennes (Vmoy_FR) de fréquence respiratoire,
   - des variables (x₁, x₂, ..., xₙ) représentatives du profil sociodémographique du patient et
   - le ou lesdits modèles mathématiques.
- les moyens de mémorisation sont conçus pour mémoriser en outre :
   - les valeurs de moyenne de fréquence respiratoire (FR_moy) et les durées de respiration (Dresp) et/ou
   - les moyennes de durée de respiration (Vmoy_Dresp) et les valeurs moyennes (Vmoy _FR) de fréquence respiratoire.
- il comprend des moyens de fourniture d'énergie électrique alimentant les moyens de mémorisation et ledit au moins un processeur, de préférence les moyens de fourniture d'énergie électrique comprennent une ou plusieurs batteries.
- les variables (x₁, x₂, ..., xₙ) représentatives du profil sociodémographique du patient sont choisies parmi :
   i) l'âge, le poids, la taille, l'indice de masse corporelle (IMC) et le sexe du patient,
   ii) l'état tabagique du patient, i.e., fumeur ou non fumeur,
   iii) le type de dispositif d'assistance respiratoire utilisé par le patient, i.e., CPAP, LOX...,
   iv) le lieu de résidence du patient (i.e. agglomération, zone rurale...),
   v) statut marital (i.e. en couple ou personne vivant seule),
   vi) débit d'oxygène prescrit par le médecin du patient.
- la durée donnée dt est comprise entre 15 minutes et 120 minutes, de préférence entre 30 minutes et 90 minutes, typiquement de l'ordre de 60 min.
- la durée longue dL est d'au moins 6 h, de préférence d'au moins 12 h et d'au plus 48 h, de préférence encore d'au moins 18 h et d'au plus 36 h, typiquement de l'ordre de 24 h.
- la durée longue dL est supérieure à la durée donnée dt (dL>dt).
- le processeur est configuré pour répéter tout ou partie des opérations a) à e) pour obtenir plusieurs valeurs moyennes de durée de respiration (Vmoy_Dresp) et plusieurs valeurs moyennes(Vmoy_FR) de fréquence respiratoire, sur une durée de 3 à 20 jours, de préférence entre 5 et 12 jours, de préférence encore entre 6 et 10 jours, typiquement de l'ordre de 7 jours.
- il comprend un dispositif d'affichage de données configuré pour afficher au moins une alerte émise par ledit au moins un processeur en cas de détection par ledit au moins un processeur, d'un état de santé du patient correspondant à une prédiction d'exacerbation.
- le dispositif d'affichage de données comprend un écran, tel un écran d'ordinateur, de tablette ou de téléphone.
- il comprend un premier processeur, un deuxième processeur et un troisième processeur.
- au moins deux processeurs sont agencés au sein d'un même boitier, et au moins un bus de communication est agencé entre lesdits processeurs.
- il comprend un boitier comprenant ledit ou les microprocesseur(s) et un passage de gaz et un capteur de pression agencé sur ledit passage de gaz de manière à opérer des mesures de pression de gaz au sein dudit passage de gaz.
- le modèle mathématique de référence mémorisé par les moyens de mémorisation comprend un ou plusieurs arbres de décision DT dont les contributions sont agrégées pour obtenir une décision plus robuste afin de distinguer les deux états du patient, à savoir un état de santé normale ou état « Y1 », et un état de détérioration caractérisant une exacerbation, par exemple de BPCO, ou état « Y2 ». Les paramètres de ce modèle sont des seuils S = {s1, s2, ...} estimés sur chaque composante du vecteur multidimensionnel pour construite l'(ou les) arbres de décision DT noeud par noeud. Le (ou les) processeur est configuré avec une phase d'apprentissage permettant de calculer les seuils optimaux pour chaque variable à partir d'un échantillon de *n* vecteurs {X₁, X₂, ...Xₙ}. Chaque vecteur Xt est associé à l'un des deux états Y1 ou Y2 connu à priori. L'échantillon de données est collecté à partir d'un historique d'un à plusieurs mois de suivi patient, typiquement 12 mois.
- le modèle mathématique de référence déterminé au sein d'un serveur distant comprenant le troisième processeur P3.
- le processeur est configuré pour émettre une alerte lorsque qu'on prédit, c'est-à-dire détecte plus tôt, un risque un d'exacerbation par comparaison du vecteur Xt, acquis à la date t (par exemple toutes les 24h), au modèle mathématique, c'est-à-dire lorsque toutes les composantes/variables de Xt comparées à leurs seuils respectifs mémorisés conduisent dans l'état Y2 caractérisant la détérioration de l'état du patient vers une exacerbation de BPCO.
- l'alerte est donnée à partir d'une combinaison des décisions obtenues à partir de l'agrégation de plusieurs arbres DT, entre 10 et 100, typiquement 50. Cette combinaison est une moyenne simple ou pondérée avec des coefficients sur les arbres de décision DT. Les coefficients optimaux sont calculés à l'aide de techniques de type Gradient Boosting, comme décrit par Friedman, J. H. « Stochastic Gradient Boosting », 1999, pondérant la contribution de chaque prédicteur de façon à obtenir la meilleure performance.
- le boitier comprend un dispositif lumineux, de préférence au moins une lampe de type LED, permettant d'émettre un signal lumineux sur site, c'est-à-dire localement.
- il comprend des moyens d'émission de données permettant de transmettre à distance une alerte, par exemple destinée à un professionnel de santé. Par exemple, les moyens d'émission de données par radio fréquence.
- ledit au moins un processeur (P1, P2) est configuré pour créer ledit au moins un vecteur multidimensionnel (Xt*)* à partir avec les variables (x₁, x₂, ..., xₙ) représentatives du profil sociodémographique du patient, les variables statistiques (v₁, v₂, ..., vₘ) issues des moyennes de durées de respiration (Vmoy_Dresp) et de fréquence respiratoire (Vmoy_FR), et des informations (z₁, z₂, ..., zₚ) d'historique médical, tel que durée depuis la dernière exacerbation et nombre d'exacerbations passées, ainsi que des signaux vitaux (w₁, w₂, ... w_{q}) du patient qui peuvent être mesurés par des dispositifs médicaux dédiés, tels que température corporelle, pression artérielle, l'évolution du poids, la saturation en oxygène (SpO2), volume expiratoire maximum FEV1 ...
- optionnellement, il comprend aussi un accéléromètre permettant de déterminer l'activité physique du patient (repos, déambulation etc...), laquelle est alors prise en compte pour définir le vecteur multidimensionnel Xt.

L'invention concerne aussi une installation de suivi de patient permettant de prédire une exacerbation d'un patient atteint d'une maladie respiratoire chronique, en particulier d'une BPCO, lequel patient est traité par oxygénothérapie, en particulier à son domicile, caractérisée en ce qu'elle comprend une source de gaz reliée fluidiquement à une interface respiratoire de distribution de gaz au patient de manière à alimenter ladite interface respiratoire de distribution de gaz avec du gaz délivré par la source de gaz, et un système de traitement de données selon l'invention.

Selon le cas, l'installation de suivi de patient de l'invention peut comprendre l'une ou plusieurs des caractéristiques techniques suivantes :
- le boitier comprend des moyens d'émission de données conçus pour transmettre des données via un dispositif de transmission intermédiaire configuré pour relayer les données vers un serveur distant, de préférence le dispositif de transmission intermédiaire comprend un ordinateur ou un modem, en particulier un modem GSM.
- le boitier comprend des moyens d'émission conçus pour transmettre des données par radiofréquence ou par Bluetooth™.

L'invention va maintenant être mieux comprise grâce à la description détaillée suivante, faite à titre illustratif mais non limitatif, en référence aux figures annexées parmi lesquelles :
- la Figure 1 schématise un mode de réalisation du principe de fonctionnement général d'une installation médicale permettant de suivre à distance un patient traité par oxygénothérapie à son domicile ;
- les Figure 2a et 2b illustrent l'augmentation de la fréquence respiratoire et la durée de respiration dans les jours précédents une crise d'exacerbation de BPCO,
- la Figure 3 schématise une installation de suivi de patient selon l'invention, laquelle intègre le boitier de la Figure 4,
- la Figure 4 représente un mode de réalisation schématique d'un système selon l'invention, et
- Figure 5 illustre le traitement de données respiratoires pour extraire les variables représentatives de l'état du patient à partir des fréquences respiratoires et durées de traitement acquises sur plusieurs jours.

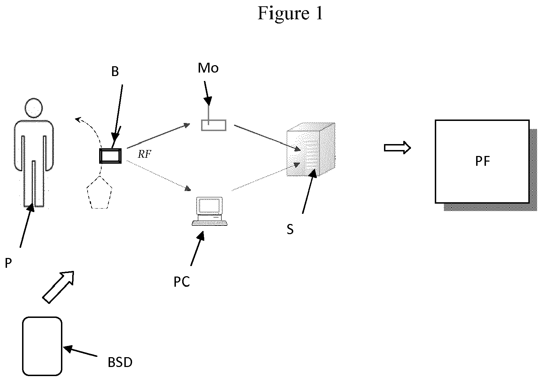

La Figure 1 schématise un mode de réalisation du principe de fonctionnement général d'une installation médicale permettant de suivre à distance, et dès lors de prédire ou détecter au plus tôt, une crise d'exacerbation d'un patient P atteint d'une maladie respiratoire chronique, en particulier d'une BPCO, lequel patient P est traité par oxygénothérapie à son domicile, de manière à éviter ou à réduire la fréquence de ré-hospitalisation de ce patient.

L'installation de suivi comprenant un dispositif d'acquisition et de transmission de données se présentant sous la forme d'un boitier B incluant tout ou partie d'un système de traitement de données selon la présente invention.

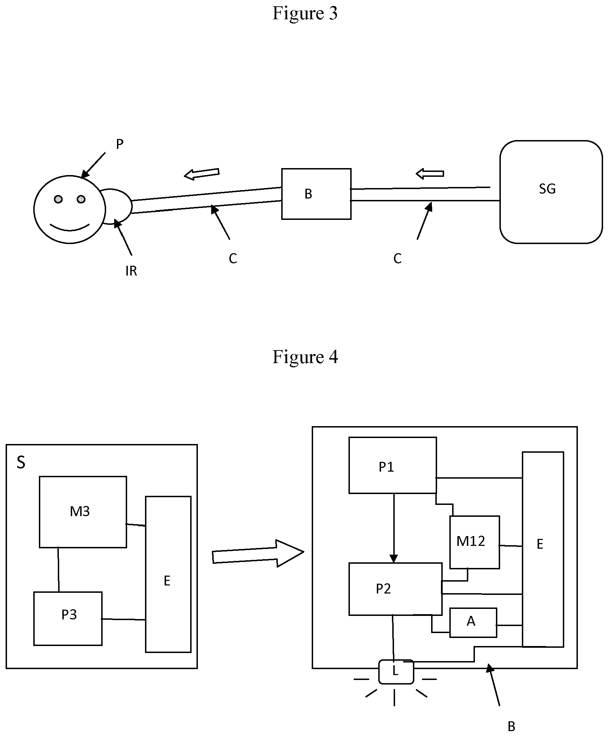

Le boitier B est intercalé, tel que schématisé en Figure 3, sur le trajet du gaz servant au traitement d'oxygénothérapie du patient P, c'est-à-dire typiquement entre une source de gaz d'oxygénothérapie SG délivrant typiquement de l'oxygène ou un gaz riche en oxygène, i.e. >80% en vol d'O₂, tel un concentrateur d'oxygène, et une interface respiratoire IR fournissant le gaz au patient 30, comme des canules nasales ou un masque respiratoire.

Ainsi, le boitier B est préférentiellement raccordé fluidiquement aux conduits C de gaz, de préférence des tuyaux flexibles ou analogues, servant à véhiculer le gaz depuis la source de gaz SG jusqu'à l'interface respiratoire IR.

Le boitier B forme une carcasse externe au sein de laquelle sont agencés un ou plusieurs capteurs de pression de gaz agencé(s) de manière à opérer des mesures de pression au sein d'un conduit de gaz traversant le boitier B et alimenté par la source SG de gaz, un ou plusieurs microprocesseurs P1, P2, mettant en oeuvre une (ou plusieurs) méthode de calcul (ou algorithme) qui reçoit les signaux de mesure de pression provenant du ou des capteurs de pression et qui les traite pour en déduire des données respiratoires (fréquences, durée de respiration ...), des moyens de mémorisation M12, telle une mémoire, éventuellement des moyens d'émission à distance des données issues du microprocesseur, et une source d'énergie E électrique, telle une batterie rechargeable ou non.

La (ou les) source d'énergie E électrique alimente en courant électrique les différents composants nécessitant de l'énergie électrique pour fonctionner, notamment les capteur(s), le(s) microprocesseur(s) P1, P2, les moyens de mémorisation M12 et éventuellement les moyens d'émission de données, par exemple une antenne radiofréquence RF, de manière à permettre à l'ensemble de fonctionner en autonomie.

Le rôle des microprocesseurs P1, P2, P3... sera expliqué ultérieurement.

L'architecture interne du boitier B varie selon le mode de réalisation considéré.

Ainsi, la Figure 4 représente un mode de réalisation, dans lequel le boitier B comprend un premier processeur P1 et un deuxième processeur P2 agencés au sein dudit boitier B, en étant reliés par un bus de communication (non montré).

Le deuxième processeur P2 est connecté aux moyens de mémorisation M12, telle une puce mémoire (chips) ou analogue. Préférentiellement, le premier processeur P1 peut aussi être connecté aux moyens de mémorisation M12. Le premier processeur P1, le deuxième processeur P2 et leurs moyens de mémorisation M12 sont alimentés en énergie par la source d'énergie E électrique.

Le boitier B peut aussi comprendre un dispositif lumineux L, de préférence au moins une lampe de type LED, permettant d'alerter l'utilisateur, comme expliqué ci-après. Par ailleurs, le boitier B peut aussi comprendre un afficheur A de données, tel un écran digital. Le dispositif lumineux L et/ou l'afficheur A sont aussi alimentés en électricité par la source d'énergie E électrique.

Par ailleurs, le troisième processeur P3 se trouve au sein d'un serveur S distant qui communique à distance avec le boitier B grâce aux moyens d'émission de données. Le serveur S peut être connecté à un afficheur de données, tel un écran d'ordinateur ou autre permettant à une personne de prendre connaissance des informations transmises au serveur S par le boitier B et/ou de données traitées au sein du serveur S, y compris d'alarmes.

Au sein du serveur S, l'historique des données collectées par le boitier B est transmis et mémorisé dans les moyens de mémorisation M3 pour constituer et/ou enrichir une base d'apprentissage c'est-à-dire échantillon de données servant à calculer le modèle mathématique par le processeur P3.

Le troisième processeur P3 est alimenté en courant électrique par une source de courant E, comme une batterie, le secteur électrique ou autre, et est par ailleurs relié à une mémoire de stockage M3 de données.

La présente invention permet une surveillance à distance d'un ou de plusieurs patients soignés à domicile, grâce à la transmission en temps-réel de données respiratoires par le boitier B, au serveur S distant, tel un serveur ASIP (hébergement agréé dans le domaine de la Santé), comme illustré en Figure 1. La transmission des données vers le serveur distant 1 peut être opérée via un ordinateur PC ou un modem Mo, par exemple de type GSM.

En d'autres termes, le boitier B indépendant opère d'abord des mesures de pression P du flux de gaz d'oxygénothérapie circulant, pendant une durée donnée dt, dans le passage ou conduit traversant le boitier B de manière à pouvoir en déduire, c'est-à-dire déterminer, à l'aide du microprocesseur P1, des variations de pression Dp du flux de gaz pendant la durée donnée dt, à partir de ces mesures de pression P. La durée donnée dt peut être typiquement de l'ordre de 1 heure.

Les mesures de pression sont opérées par un (ou plusieurs) capteurs de pression qui transmettent les signaux de mesure au microprocesseur P1 où ils sont traités.

Ces variations de pression DP pendant ladite durée donnée dt permettent au microprocesseur P1 de déterminer ensuite une durée de respiration Dresp du patient, pendant la durée donnée dt, et d'en déduire au moins une valeur de fréquence respiratoire Val_FR pendant la durée donnée dt.

Cette opération est répétée plusieurs fois pendant la durée de respiration Dresp de manière à obtenir plusieurs valeurs de fréquences respiratoires Val_FR successives.

En fait, la durée de respiration Dresp correspond à la durée, par exemple 50 minutes, pendant la période de temps dt considérée, par exemple une durée dt de 1 heure, pendant laquelle le patient a inhalé du gaz. La durée de respiration Dresp est en fait formée d'une succession d'intervalles I₁, I₂, I₃, ... Iₙ de temps, par exemple des intervalles de 1 à 5 minutes, qui peuvent être prédéfinis ou préconfigurés, notamment dans le microprocesseur, pendant lesquels le patient a respiré du gaz, par exemple de l'oxygène. Plusieurs fréquences respiratoires FR peuvent donc être mesurées pendant ces intervalles de temps, donc pendant la durée totale Dresp, de manière à déterminer plusieurs valeurs de fréquence respiratoire Val_FR successives correspondant auxdits intervalles I₁, I₂, I₃, ... Iₙ de temps. En effet, la fréquence respiratoire FR n'est pas constante pendant toute la durée de respiration Dresp. Ces valeurs de fréquence respiratoire Val_FR successives vont être utilisées ensuite par le microprocesseur P1 comme expliqué ci-après.

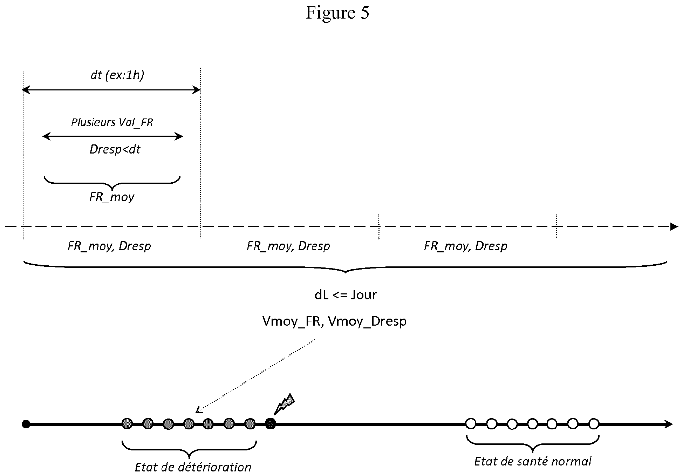

Le microprocesseur P1 calcule alors au moins une valeur moyenne de fréquence respiratoire FR_moy sur la durée de respiration Dresp, à partir desdites plusieurs valeurs de fréquences respiratoires Val_FR successives mesurées pendant la durée de respiration Dresp. Ensuite, le premier processeur P1 calcule au moins une valeur moyenne de durée de respiration Vmoy_Dresp à partir de plusieurs durées de respiration Dresp successives, pendant une durée longue dL de maximum 24 heures. Ceci est schématisé sur la Figure 5 qui illustre le traitement des données respiratoires par le processeur P1 (cf. a) à g) ci-avant).

Le premier processeur P1 détermine, en outre, à partir de plusieurs valeurs moyennes des données respiratoires obtenues sur la durée longue dL, de préférence sur plusieurs jours, des variables de coefficient linéaire et de somme à partir des valeurs moyennes de durées de respiration (Vmoy_Dresp) et variables correspondantes au coefficient linéaire, la médiane, la variance et l'auto-corrélation des valeurs moyennes (Vmoy_FR) de fréquence respiratoire. Celles-ci représentent les variables statistiques (v₁, v₂, ..., vₘ) caractérisant la dynamique d'évolution de l'état de santé du patient.

Les moyens de mémorisation M12 sont en fait conçus pour mémoriser les valeurs de moyenne de fréquence respiratoire _FR_moy, les durées de respiration Dresp successives fournies par le processeur P1, mais aussi d'autres données comme les valeurs moyennes de durée de respiration Vmoy_Dresp et Vmoy_FR, les variables statistiques (v₁, v₂, ..., vₘ) estimés sur plusieurs jours et aussi un ou plusieurs modèles mathématiques prédéfinis représentatifs de l'état de santé.

Ensuite, le système sélectionne, au travers du processeur P2, au sein d'au moins une base de données, plusieurs variables (x₁, x₂, ..., xₙ) représentatives du profil sociodémographique du patient mémorisées au sein de ladite au moins une base de données et les aligne avec les variables statistiques (v₁, v₂, ..., vₘ) caractérisant la dynamique d'évolution de l'état de santé du patient pour constituer un vecteur multidimensionnel Xt à chaque date t.

Ainsi, le processeur P2 compare à chaque date le vecteur multidimensionnel Xt à un ou plusieurs modèles mathématiques prédéfinis et mémorisés de manière à détecter une dégradation de l'état de santé du patient P et prédire une crise imminente d'exacerbation de BPCO.

En particulier, les variables (x₁, x₂, ..., xₙ) représentatives du profil sociodémographique du patient sont choisies notamment parmi :
- l'âge, le poids, la taille, l'indice de masse corporelle et le sexe du patient,
- l'état tabagique du patient,
- le type de dispositif d'assistance respiratoire utilisé par le patient,
- le lieu de résidence du patient,
- statut marital,
- débit d'oxygène prescrit par le médecin du patient.

Toutefois, le système permet aussi d'utiliser des données supplémentaires notamment l'historique médical d'un patient qui peut être disponible dans une base de données et lequel comprend des données patient du type durée depuis la dernière exacerbation et nombre d'exacerbations passées, ainsi que des signaux vitaux du patient qui peuvent être mesurés par des dispositifs médicaux dédiés, tel que température corporelle, pression artérielle, évolution du poids, saturation en oxygène (SpO2), le volume expiratoire maximum (FEV1)...

Ainsi, l'un des (ou les) processeurs P1, P2 peut être (sont) configuré pour créer le vecteur multidimensionnel (Xt) à partir avec les variables (x₁, x₂, ..., xₙ) représentatives du profil sociodémographique du patient, les variables statistiques (v₁, v₂, ..., vₘ) issues des moyennes de durées de respiration (Vmoy_Dresp) et de fréquence respiratoire (Vmoy_FR), et des informations (z₁, z₂, ..., zₚ) d'historique médical tel que la durée depuis la dernière exacerbation et nombre d'exacerbations passées, ainsi que des signaux vitaux (w1, w2, ...wq) du patient qui peuvent être mesurés par des dispositifs médicaux dédiés, tels que température corporelle, pression artérielle, l'évolution du poids, la saturation en oxygène (SpO2), le volume expiratoire maximum FEV1 ...

Le ou lesdits modèles mathématiques sont estimés à partir de données correspondant à des états de santé normaux et à des états pré-exacerbatoires. Il s'ensuit que le système déduit alors de la comparaison avec le ou lesdits modèles mathématiques, l'état de santé du patient considéré de manière à prédire une exacerbation imminente.

Lorsque, pour un patient donné, le système détecte une dégradation de l'état de santé du patient P susceptible de conduire à une crise imminente d'exacerbation de BPCO, le processeur P2 est configuré pour émettre une alerte, telle une alarme visuelle ou sonore, par exemple l'activation d'un dispositif lumineux L, de manière à avertir de la détection d'une telle dégradation de l'état de santé du patient P et donc d'une crise imminente possible d'exacerbation de BPCO.

Des moyens de fourniture d'énergie électrique E, telle une ou des batteries ou analogue, alimentent les différents composants du système, en particulier les moyens de mémorisation M3, M12 et les processeurs P1, P2, P3.

Le mode de fonctionnement du système selon la présente invention est particulièrement efficace pour détecter l'imminence d'une exacerbation de BPCO, i.e. une crise de BPCO.

En effet, des essais réalisés, dans le cadre de la présente invention, sur des patients souffrant de BPCO et traités par oxygénothérapie ont montré qu'une augmentation de l'utilisation des équipements de traitement d'oxygénothérapie par les patients sous oxygénothérapie, donc de la durée de respiration Dresp du patient, est, dans une grande majorité des cas, un signe précurseur de l'avènement d'une exacerbation, comme illustré en Figure 2a et 2b.

Ainsi, les Figures 2a et 2b montrent clairement que la fréquence respiratoire FR et la durée de respiration Dresp, augmentent dans les jours précédents une crise d'exacerbation de BPCO (à droite sur la Fig. 2a) par rapport à une situation normale (à gauche sur la Fig. 2a) car le patient tente de compenser une capacité respiratoire insuffisante en utilisant plus fréquemment son dispositif de traitement par oxygénothérapie. En d'autres termes, il existe un lien de corrélation entre usage accru d'un équipement de traitement d'oxygénothérapie et crise d'exacerbation de BPCO.

Les Figures 2b conduisent aux mêmes constatations illustrées par les courbes de tendance reflétant l'évolution de la fréquence respiratoire FR et la durée de respiration du patient Dresp sur une durée d'observation de 20 jours. On y constate que, là aussi, la fréquence respiratoire FR augmente sensiblement et la durée de respiration Dresp augmente fortement en cas d état pré-exacerbatoire (courbes de droite sur Fig. 2b) par rapport à un état normal du patient (courbes de gauche sur Fig. 2b).

Préférentiellement, comme déjà évoqué, le premier processeur P1 calcule des valeurs de Vmoy_FR et Vmoy_Desp et leurs dérivées statistiques (v₁, v₂, ..., vₘ) à partir des mesures de pression, alors que le deuxième processeur P2 récupère les variables respiratoires (i.e., des données dynamiques) et les croisent avec les données sociodémographiques (i.e., des données statiques) comprenant des variables (x₁, x₂, ..., xₙ) représentatives du profil sociodémographique du patient et optionnellement d'autres données patient, telles que des signaux vitaux (T, tension artérielle...) ou des données médicales (nombre de crises d'exacerbation passées...), qui sont mémorisées par la mémoire M12 du boitier B de la Figure 4 par exemple.

Les données sociodémographiques proviennent par exemple d'une base de données sociodémographiques BSD (cf. Figure 1) communiquant avec le système de l'invention, notamment avec le boitier B et le serveur S.

Toutes ces données permettent d'établir un vecteur multidimensionnel Xt et le modèle mathématique, dont les paramètres sont aussi stockés dans la mémoire M12, est alors comparé avec le vecteur multidimensionnel Xt, à chaque instant ou date t de manière à pouvoir détecter les exacerbations BPCO et envoyer alors un signal d'alerte servant à avertir le personnel de santé (médecin...) et/ou le patient lui-même.

Par ailleurs, le troisième processeur P3 permet quant à lui d'estimer les paramètres du modèle mathématique sur un échantillon de plusieurs vecteurs multidimensionnels collectés sur un historique de 1 à 12 mois. Les paramètres optimaux sont alors estimés pendant une phase d'apprentissage et transférés et stockés dans la mémoire M12 pour permettre au deuxième processeur P2 de déterminer si une exacerbation est proche ou non. Les paramètres du modèle mathématique sont mis à jour régulièrement par l'apprentissage incrémental ou périodique.

Le troisième processeur P3 est préférentiellement situé sur un serveur S distant qui comprend par ailleurs des moyens de mémorisation M3 et de calcul plus importants que le boitier B où sont agencés les premier et deuxième processeurs P1, P2 et la mémoire M12.

Les échanges de données entre le boitier B et le serveur distant S de la Figure 4 sont opérés à distance, via un ordinateur PC relié à internet, un modem Mo, par exemple de type GSM, ou autre.

Le système de traitement de données selon l'invention, tel que détaillé ci-avant, permet de prédire une crise d'exacerbation d'un patient atteint d'une maladie respiratoire chronique, en particulier d'une BPCO, lequel patient est traité par oxygénothérapie, en particulier à son domicile, et d'alerter ensuite le personnel soignant ou analogue de manière à éviter ou à réduire la fréquence des ré-hospitalisations dudit patient.

## Revendications

1. Système de traitement de données permettant de prédire une crise d'exacerbation d'un patient atteint d'une maladie respiratoire chronique, en particulier d'une BPCO, et traité par oxygénothérapie, comprenant au moins un processeur (P1, P2) configuré pour :
a) déterminer des variations de pression (DP) de flux de gaz pendant une durée donnée (dt), à partir de mesures de pression (P) d'un flux de gaz d'oxygénothérapie administré à un patient pendant la durée donnée (dt),
b) déterminer une durée de respiration (Dresp) du patient, avec Dresp < dt, pendant la durée donnée dt, à partir des variations de pression (DP) pendant ladite durée donnée (dt), ladite durée de respiration (Dresp) étant définie comme la durée, pendant la période de temps dt considérée, pendant laquelle le patient a inhalé du gaz, déduire des mesures de pression (P) au moins une valeur de fréquence respiratoire (Val_FR) pendant la durée donnée dt et répéter plusieurs fois cette détermination de valeur de fréquence respiratoire pendant la durée de respiration (Dresp) de manière à obtenir plusieurs valeurs de fréquences respiratoires (Val_FR) successives mesurées pendant la durée de respiration (Dresp),
c) calculer au moins une valeur moyenne de fréquence respiratoire (FR_moy) sur la durée de respiration (Dresp), à partir de plusieurs valeurs de fréquences respiratoires (Val_FR) successives mesurées pendant la durée de respiration (Dresp),
d) calculer au moins une valeur moyenne de durée de respiration (Vmoy_Dresp) à partir de plusieurs durées de respiration (Dresp) successives pendant une durée longue dL,
e) déterminer, à partir de plusieurs valeurs moyennes de fréquence respiratoire (FR_moy) obtenues sur la durée longue (dL), une valeur moyenne (Vmoy_FR) de fréquence respiratoire,
f) répéter toute ou partie des opérations opérées en a) à e) pour obtenir plusieurs valeurs moyennes de durée de respiration (Vmoy_Dresp) et plusieurs valeurs moyennes (Vmoy _FR) de fréquence respiratoire, sur une durée de plusieurs jours,
g) calculer à partir des valeurs moyennes de durée de respiration (Vmoy_Dresp) et des valeurs moyennes (Vmoy_FR) de fréquence respiratoire obtenues sur la durée de plusieurs jours, plusieurs variables statistiques (v₁, v₂, ..., vₘ) correspondant au moins aux valeurs :
- de coefficient linéaire et de la somme des valeurs moyennes de durées de respiration (Vmoy_Dresp).
- de coefficient linéaire, de la valeur médiane, de la variance et de l'auto-corrélation estimées à partir des valeurs moyennes (Vmoy_FR) de fréquence respiratoire,
h) sélectionner au sein d'au moins une base de données, plusieurs variables (x₁, x₂, ..., xₙ) représentatives du profil sociodémographique du patient mémorisées au sein de ladite au moins une base de données,
i) comparer à chaque date t prédéfinie, un vecteur multidimensionnel Xt, représentant au moins les variables statistiques (v₁, v₂, ..., vₘ) issues des données respiratoires et les variables (x₁, x₂, ..., xₙ) représentatives du profil sociodémographique du patient, à un ou plusieurs modèles mathématiques prédéfinis et mémorisés, le ou lesdits modèles mathématiques étant estimés à partir de données correspondant à des états de santé normaux et à des états pré-exacerbatoires,
j) et déduire de la comparaison avec le ou lesdits modèles mathématiques, l'état de santé du patient considéré de manière à prédire une exacerbation.

2. Système selon la revendication 1, **caractérisé en ce qu'**il comprend des moyens de mémorisation (M) conçus pour mémoriser :
- les variables statistiques (v₁, v₂, ..., vₘ) obtenues à partir des valeurs moyennes de durée de respiration (Vmoy_Dresp) et les valeurs moyennes (Vmoy_FR) de fréquence respiratoire,
- les variables (x₁, x₂, ..., xₙ) représentatives du profil sociodémographique du patient,
- et le ou lesdits modèles mathématiques.

3. Système selon la revendication 2, **caractérisé en ce que** les moyens de mémorisation (M) sont conçus pour mémoriser en outre les valeurs de moyenne de fréquence respiratoire (FR_moy) et les durées de respiration (Dresp) et/ou les moyennes de durée de respiration (Vmoy_Dresp) et les valeurs moyennes (Vmoy_FR) de fréquence respiratoire.

4. Système selon l'une des revendications précédentes, **caractérisé en ce qu'**il comprend des moyens de fourniture d'énergie électrique (E) alimentant les moyens de mémorisation (M) et ledit au moins un processeur (P1, P2).

5. Système selon la revendication 1 ou 3, **caractérisé en ce que** les variables (x₁, x₂, ..., xₙ) représentatives du profil sociodémographique du patient sont choisies parmi :
i) l'âge, le poids, la taille, l'indice de masse corporelle et le sexe du patient,
ii) l'état tabagique du patient,
iii) le type de dispositif d'assistance respiratoire utilisé par le patient,
iv) le lieu de résidence du patient,
v) statut marital,
vi) débit d'oxygène prescrit par le médecin du patient.

6. Système selon la revendication 1, **caractérisé en ce que** la durée donnée dt est comprise entre 15 minutes et 120 minutes, typiquement de l'ordre de 60 min, ou la durée longue dL est d'au moins 6 h, typiquement de l'ordre de 24 h, avec dL > dt.

7. Système selon la revendication 1, **caractérisé en ce que** le processeur (P1, P2) est configuré pour répéter tout ou partie des opérations a) à e) pour obtenir plusieurs valeurs moyennes de durée de respiration (Vmoy_Dresp) et plusieurs valeurs moyennes(Vmoy_FR) de fréquence respiratoire, sur une durée de 3 à 20 jours, typiquement de l'ordre de 7 jours.

8. Système selon l'une des revendications précédentes, **caractérisé en ce qu'**il comprend un dispositif d'affichage de données (A) configuré pour afficher au moins une alerte émise par ledit au moins un processeur (P1, P2) en cas de détection par ledit au moins un processeur (P1, P2), d'un état de santé du patient correspondant à une prédiction d'exacerbation.

9. Système selon l'une des revendications précédentes, **caractérisé en ce qu'**il comprend un premier processeur (P1), un deuxième processeur (P2) et un troisième processeur (P3).

10. Système selon l'une des revendications précédentes, **caractérisé en ce qu'**il comprend un boitier (B) comprenant ledit au moins un microprocesseur (P1, P2) et un passage de gaz et un capteur de pression agencé sur ledit passage de gaz de manière à opérer des mesures de pression de gaz au sein dudit passage de gaz.

11. Système selon l'une des revendications précédentes, **caractérisé en ce que** ledit au moins un processeur (P1, P2) est configuré pour émettre une alerte lorsque, pour un patient donné, une exacerbation est prédite, et de préférence pour afficher une information d'état de santé du patient.

12. Système selon la revendication 2, **caractérisé en ce que** ledit au moins un processeur (P1, P2) est configuré pour créer ledit au moins un vecteur multidimensionnel (Xt) à partir avec les variables (x₁, x₂, ..., xₙ) représentatives du profil sociodémographique du patient, les variables statistiques (v₁, v₂, ..., vₘ) issues des moyennes de durées de respiration (Vmoy_Dresp) et de fréquence respiratoire (Vmoy_FR), et des informations (z₁, z₂, ..., zₚ) d'historique médical et des signaux vitaux (w1, w2, ...wq) du patient.

13. Installation de suivi de patient permettant de prédire une exacerbation d'un patient atteint d'une maladie respiratoire chronique, en particulier d'une BPCO, lequel patient est traité par oxygénothérapie, en particulier à son domicile, **caractérisée en ce qu'**elle comprend une source de gaz reliée fluidiquement à une interface respiratoire de distribution de gaz au patient de manière à alimenter ladite interface respiratoire de distribution de gaz avec du gaz délivré par la source de gaz, et un système de traitement de données selon l'une des revendications précédentes.

## Patentansprüche

1. Datenverarbeitungssystem zur Vorhersage einer akuten Zustandsverschlimmerung eines Patienten, der an einer chronischen Atemwegserkrankung, insbesondere an einer COPD, leidet, und mit einer Sauerstofftherapie behandelt wird, das mindestens einen Prozessor (P1, P2) umfasst, der konfiguriert ist zum:
a) Bestimmen der Druckschwankungen (DP) einer Gasströmung während einer gegebenen Dauer (dt) aus Druckmessungen (P) einer einem Patienten während der gegebenen Dauer (dt) verabreichten Gasströmung zur Sauerstofftherapie,
b) Bestimmen einer Atemdauer (Dresp) beim Patienten, mit Dresp < dt, während der gegebenen Dauer dt aus den Druckschwankungen (DP) während der gegebenen Dauer (dt), wobei die Atemdauer (Dresp) definiert ist als die Dauer, während des betrachteten Zeitraums dt, während derer der Patient Gas eingeatmet hat, Ableiten aus den Druckmessungen (P) mindestens eines Atemfrequenzwertes (Val_FR) während der gegebenen Dauer dt und mehrfaches Wiederholen dieser Bestimmung des Atemfrequenzwertes während der Atemdauer (Dresp), um mehrere aufeinanderfolgende Atemfrequenzwerte (Val_FR), die während der Atemdauer (Dresp) gemessen wurden, zu erhalten,
c) Berechnen mindestens eines Atemfrequenz-Mittelwertes (FR_moy) über die Atemdauer (Dresp), aus mehreren aufeinanderfolgenden Atemfrequenzwerten (Val_FR), die während der Atemdauer (Dresp) gemessen wurden,
d) Berechnen mindestens eines Atemdauer-Mittelwertes (Vmoy_Dresp) aus mehreren aufeinanderfolgenden Atemdauer (Dresp) während einer langen Dauer dL,
e) Bestimmen aus mehreren Atemfrequenz-Mittelwerten (FR_moy), die während der langen Dauer (dL) erhalten wurden, eines Mittelwertes (Vmoy_FR) der Atemfrequenz,
f) Wiederholen aller oder eines Teils der unter a) bis e) durchgeführten Vorgänge, um mehrere Atemdauer-Mittelwerte (Vmoy_Dresp) und mehrere Mittelwerte (Vmoy_FR) der Atemfrequenz über eine Dauer von mehreren Tagen zu erhalten,
g) Berechnen aus den Atemdauer-Mittelwerten (Vmoy_Dresp) und den über die Dauer von mehreren Tagen erhaltenen Mittelwerten (Vmoy_FR) der Atemfrequenz mehrerer statistischer Variablen (v₁, v₂, ..., vₘ), die mindestens den folgenden Werten entsprechen:
- Linearkoeffizient und die Summe der Atemdauer-Mittelwerte (Vmoy_Dresp).
- Linearkoeffizient, der Medianwert, die Varianz und die Autokorrelation, die aus den Mittelwerten (Vmoy_FR) einer Atemfrequenz geschätzt werden,
h) Auswählen aus mindestens einer Datenbank mehrerer in der mindestens einen Datenbank gespeicherter Variablen (x₁, x₂, ..., xₙ), die das soziodemografische Profil des Patienten repräsentieren,
i) Vergleichen mit jedem vordefinierten Datum t, eines mehrdimensionalen Vektors Xt, der mindestens die statistischen Variablen (v₁, v₂, ..., vₘ) aus den Atmungsdaten und der Variablen (x₁, x₂, ..., xₙ), die das soziodemografische Profil des Patienten repräsentieren, mit einem oder mehreren vordefinierten und gespeicherten mathematischen Modellen, wobei das oder die mathematische(n) Modell(e) aus Daten geschätzt wird/werden, die normalen Gesundheitszuständen und Zuständen vor der Verschlimmerung entsprechen,
j) und Ableiten des Gesundheitszustandes des betreffenden Patienten aus dem Vergleich mit dem oder den genannten mathematischen Modell(en), um eine Verschlimmerung vorherzusagen.

2. System nach Anspruch 1, **dadurch gekennzeichnet, dass** es Speichermittel (M) umfasst, die zum Speichern bestimmt sind von:
- den statistischen Variablen (v₁, v₂, ..., vₘ), die aus den Atemdauer-Mittelwerten (Vmoy_Dresp) und den Mittelwerten (Vmoy_FR) einer Atemfrequenz erhalten wurden,
- den Variablen (x₁, x₂, ..., xₙ), die das soziodemografische Profil des Patienten repräsentieren,
- und dem oder den mathematischen Modell(en).

3. System nach Anspruch 2, **dadurch gekennzeichnet, dass** die Speichermittel (M) dazu bestimmt sind, weiter die Atemfrequenz-Mittelwerte (FR_moy) und die Atemdauer (Dresp) und/oder die Atemdauer-Mittelwerte (Vmoy_Dresp) und die Mittelwerte (Vmoy_FR) einer Atemfrequenz zu speichern.

4. System nach einem der vorstehenden Ansprüche, **dadurch gekennzeichnet, dass** es Stromversorgungsmittel (E) umfasst, welche die Speichermittel (M) und den mindestens einen Prozessor (P1, P2) versorgen.

5. System nach Anspruch 1 oder 3, **dadurch gekennzeichnet, dass** die Variablen (x₁, x₂, ..., xₙ), die das soziodemografische Profil des Patienten repräsentieren, ausgewählt sind aus:
i) dem Alter, dem Gewicht, der Größe, dem Body-Mass-Index und dem Geschlecht des Patienten,
ii) dem Rauchstatus des Patienten,
iii) der Art der vom Patienten verwendeten Vorrichtung zur Unterstützung der Atmung,
iv) dem Wohnort des Patienten,
v) Familienstand,
vi) Sauerstoff-Volumenstrom, der vom Arzt des Patienten verschrieben ist.

6. System nach Anspruch 1, **dadurch gekennzeichnet, dass** die gegebene Dauer dt zwischen 15 Minuten und 120 Minuten, typischerweise im Bereich von 60 Minuten liegt, oder die lange Dauer dL mindestens 6 Stunden, typischerweise im Bereich von 24 Stunden liegt, mit dL > dt.

7. System nach Anspruch 1, **dadurch gekennzeichnet, dass** der Prozessor (P1, P2) konfiguriert ist, um alle oder einen Teil der Vorgänge a) bis e) zu wiederholen, um mehrere Atemdauer-Mittelwerte (Vmoy_Dresp) und mehrere Mittelwerte (Vmoy_FR) einer Atemfrequenz über eine Dauer von 3 bis 20 Tagen, typischerweise im Bereich von 7 Tagen, zu erhalten.

8. System nach einem der vorstehenden Ansprüche, **dadurch gekennzeichnet, dass** es eine Datenanzeigevorrichtung (A) umfasst, die konfiguriert ist, um mindestens einen Alarm anzuzeigen, der von dem mindestens einen Prozessor (P1, P2) bei Detektion eines Gesundheitszustands eines Patienten entsprechend einer Vorhersage zur Verschlimmerung durch den mindestens einen Prozessor (P1, P2) ausgegeben wird.

9. System nach einem der vorstehenden Ansprüche, **dadurch gekennzeichnet, dass** es einen ersten Prozessor (P1), einen zweiten Prozessor (P2) und einen dritten Prozessor (P3) umfasst.

10. System nach einem der vorstehenden Ansprüche, **dadurch gekennzeichnet, dass** es ein Gehäuse (B) umfasst, das den mindestens einen Mikroprozessor (P1, P2) und einen Gasdurchlass und einen Drucksensor umfasst, der an dem Gasdurchlass dazu angeordnet ist, Gasdruckmessungen innerhalb des Gasdurchlasses durchzuführen.

11. System nach einem der vorstehenden Ansprüche, **dadurch gekennzeichnet, dass** der mindestens eine Prozessor (P1, P2) konfiguriert ist, um einen Alarm auszugeben, wenn für einen gegebenen Patienten eine Verschlimmerung vorhergesagt ist und vorzugsweise, um eine Information zum Gesundheitszustand des Patienten anzuzeigen.

12. System nach Anspruch 2, **dadurch gekennzeichnet, dass** der mindestens eine Prozessor (P1, P2) konfiguriert ist, um den mindestens einen mehrdimensionalen Vektor (Xt) aus Variablen (x₁, x₂, ..., xₙ), die das soziodemografische Profil des Patienten repräsentieren, den statistischen Variablen (v₁, v₂, ..., vₘ) aus den Atemdauer-Mittelwerten (Vmoy_Dresp) und der Atemfrequenz (Vmoy_FR) und aus Informationen (z₁, z₂, ..., zₚ) zu der Krankengeschichte und den Vitalwerten (w1, w2, ...wq) des Patienten zu erzeugen.

13. Patientenüberwachungsanordnung zur Vorhersage einer Verschlimmerung bei einem Patienten, der an einer chronischen Atemwegserkrankung, insbesondere COPD, leidet, wobei der Patient mit Sauerstofftherapie, insbesondere bei sich zu Hause, behandelt wird, **dadurch gekennzeichnet, dass** sie eine Gasquelle, die mit einer Beatmungsschnittstelle zur Gasausgabe an den Patienten fluidisch verbunden ist, um die Beatmungsschnittstelle zur Gasausgabe mit Gas zu versorgen, das von der Gasquelle geliefert wird, und ein Datenverarbeitungssystem nach einem der vorstehenden Ansprüche.

## Claims

1. Data processing system for predicting an exacerbation crisis of a patient suffering from a chronic respiratory disease, in particular a COPD, and treated by oxygen therapy, comprising at least one processor (P1, P2) configured to:
a) determine gas flow pressure variations (DP) for a given duration (dt) from pressure measurements (P) of an oxygenotherapy gas flow administered to a patient for the given duration (dt),
b) determine a respiration duration (Dresp) of the patient, with Dresp < dt, for the given duration dt, from pressure variations (DP) during said given duration (dt), said respiration duration (Dresp) being defined as the duration, for the considered time period dt, during which the patient has inhaled gas, to deduct from pressure measurements (P) at least a respiratory frequency value (Val_FR) for the given duration dt and to repeat, several times, this determination of respiratory frequency value for the respiration duration (Dresp) so as to obtain several successive respiratory frequency values (Val_FR) measured during the respiration duration (Dresp),
c) calculate at least one average respiratory frequency value (FR_moy) over the respiration duration (Dresp), from several successive respiratory frequency values (Val_FR) measured during the respiration duration (Dresp),
d) calculate at least one average respiration duration value (Vmoy_Dresp) from several successive respiration durations (Dresp) during a long duration dL,
e) determine, from several average respiratory frequency values (FR_moy) obtained over the long duration (dL), an average respiratory frequency value (Vmoy_FR),
f) repeat all or some of the operations carried out in a) to e) to obtain several average respiration duration values (Vmoy_Dresp) and several average respiratory frequency values (Vmoy_FR), over a duration of several days,
g) calculate from the average respiration duration values (Vmoy_Dresp) and the average respiratory frequency values (Vmoy_FR) obtained over the duration of several days, several statistical variables (v₁, v₂, ..., vₘ) corresponding to at least the values:
- of linear coefficient and the sum of the average respiration duration values (Vmoy_Dresp),
- of linear coefficient, of the median value, of the variance and of the autocorrelation estimated from the average respiratory frequency values (Vmoy_FR),
h) select within at least one database, several representative variables (x₁, x₂, ..., xₙ) of the sociodemographic profile of the patient stored within said at least one database,
i) compare, at each predefined date t, a multidimensional vector Xt, representing at least the statistical variables (v₁, v₂, ..., vₘ) from respiratory data and the representative variables (x₁, x₂, ..., xₙ) of the sociodemographic profile of the patient, with one or more predefined and stored mathematical models, said mathematical model(s) being estimated from data corresponding to the normal health states and to the pre-exacerbatory states,
j) and deduce from the comparison with said mathematical model(s), the health state of the patient in question so as to predict an exacerbation.

2. System according to claim 1, **characterised in that** it comprises storage means (M) designed to store:
- the statistical variables (v₁, v₂, ..., vₘ) obtained from the average respiration duration values (Vmoy_Dresp) and the average respiratory frequency values (Vmoy_FR),
- the representative variables (x₁, x₂, ..., xₙ) of the sociodemographic profile of the patient,
- and said mathematical model(s).

3. System according to claim 2, **characterised in that** the storage means (M) are designed to furthermore store the average respiratory frequency values (FR_moy) and the respiration durations (Dresp) and/or the average respiration duration values (Vmoy_Dresp) and the average respiratory frequency values (Vmoy_FR).

4. System according to one of the preceding claims, **characterised in that** it comprises means for supplying electrical energy (E) supplying the storage means (M) and said at least one processor (P1, P2).

5. System according to claim 1 or 3, **characterised in that** the representative variables (x₁, x₂, ..., xₙ) of the sociodemographic profile of the patient are selected from among:
i) age, weight, size, body mass index and the sex of the patient,
ii) the smoking status of the patient,
iii) the type of respiratory assistance device used by the patient,
iv) the place of residence of the patient,
v) marital status,
vi) oxygen flow prescribed by the doctor of the patient.

6. System according to claim 1, **characterised in that** the given duration dt is of between 15 minutes and 120 minutes, typically of around 60 minutes, or the long duration dL is at least 6 hours, typically of around 24 hours, with dL > dt.

7. System according to claim 1, **characterised in that** the processor (P1, P2) is configured to repeat all or some of the operations a) to e) to obtain several average respiration duration values (Vmoy_Dresp) and several average respiratory frequency values (Vmoy_FR), over a duration of 3 to 20 days, typically of around 7 days.

8. System according to one of the preceding claims, **characterised in that** it comprises a data display device (A) configured to display at least one alert emitted by said at least one processor (P1, P2) in case of detection by said at least one processor (P1, P2), of a health state of the patient corresponding to an exacerbation prediction.

9. System according to one of the preceding claims, **characterised in that** it comprises a first processor (P1), a second processor (P2) and a third processor (P3).

10. System according to one of the preceding claims, **characterised in that** it comprises a casing (B) comprising said at least one microprocessor (P1, P2) and a gas passage and a pressure sensor arranged on said gas passage so as to take gas pressure measurements within said gas passage.

11. System according to one of the preceding claims, **characterised in that** said at least one processor (P1, P2) is configured to emit an alert when, for a given patient, an exacerbation is predicted, and preferably to display an item of health state information of the patient.

12. System according to claim 2, **characterised in that** said at least one processor (P1, P2) is configured to create at least one multidimensional vector (Xt) from the representative variables (x₁, x₂, ..., xₙ) of the sociodemographic profile of the patient, the statistical variables (v₁, v₂, ..., vₘ) from the average respiration durations (Vmoy_Dresp) and of respiratory frequency (Vmoy_FR), and from medical history information (z₁, z₂, ..., zₚ) and vital signals (w₁, w₂, ..., w_{q}) of the patient.

13. Patient monitoring facility making it possible to predict an exacerbation of a patient suffering from a chronic respiratory disease, in particular a COPD, which patient is treated by oxygen therapy, in particular and their home, **characterised in that** it comprises a gas source fluidically connected to a respiratory interface distributing gas to the patient so as to supply said gas distribution respiratory interface with gas delivered by the gas source, and a data processing system according to one of the preceding claims.
